(19)

Europäisches
Patentamt
European
Patent Office
Office européen
des brevets

(11) **EP 2 748 630 B1**

(12) **EUROPEAN PATENT SPECIFICATION**

(45) Date of publication and mention
of the grant of the patent:
**20.05.2015 Bulletin 2015/21**

(21) Application number: **12806140.5**

(22) Date of filing: **30.10.2012**

(51) Int Cl.:
***G01S 15/89*** *(2006.01)*      ***A61B 8/00*** *(2006.01)*

(86) International application number:
**PCT/IB2012/056003**

(87) International publication number:
**WO 2013/068883 (16.05.2013 Gazette 2013/20)**

(54) **IMPROVING LARGE VOLUME THREE-DIMENSIONAL ULTRASOUND IMAGING**

VERBESSERTE GROSSVOLUMIGE DREIDIMENSIONALE ULTRASCHALLBILDGEBUNG

AMÉLIORATION D'IMAGE ULTRASONORE TRIDIMENSIONNELLE DE GRAND VOLUME

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2011 US 201161557973 P**

(43) Date of publication of application:
**02.07.2014 Bulletin 2014/27**

(73) Proprietor: **Koninklijke Philips N.V.
5656 AE Eindhoven (NL)**

(72) Inventor: **SNYDER, Richard Allen
NL-5656 AE Eindhoven (NL)**

(74) Representative: **Steffen, Thomas
Philips
Intellectual Property & Standards
P.O. Box 220
5600 AE Eindhoven (NL)**

(56) References cited:
**WO-A1-2009/044316      WO-A1-2011/058186
GB-A- 1 262 694       US-A- 4 016 750
US-A- 4 631 710       US-A1- 2009 203 996**

## Description

### FIELD OF THE INVENTION

The present invention relates to an ultrasound system and method for providing a live three-dimensional image of a volume, for example an anatomical site of a patient. The present invention further relates to a computer program for implementing such method.

### BACKGROUND OF THE INVENTION

In three-dimensional ultrasound imaging, or volume imaging, the acquisition of a three-dimensional image is accomplished by conducting many two-dimensional scans that slice through the volume of interest. Hence, a multitude of two-dimensional images is acquired that lie next to another. By proper image processing, a three-dimensional image of the volume of interest can be built out of the multitude of two-dimensional images. The three-dimensional information acquired from the multitude of two-dimensional images is displayed in proper form on a display for the user of the ultrasound system.

Further, so-called live three-dimensional imaging, or 4D imaging, is often used in clinical applications. In live three-dimensional imaging, a real-time view on the volume can be acquired enabling a user to view moving parts of the anatomical site, for example a beating heart or else. In the clinical application of live three-dimensional imaging there is sometimes a need to image a relatively small area of the heart such as a single valve, or a septal defect, and there is sometimes the need to image a large area of the heart such as an entire ventricle.

Hence, the so-called region of interest (ROI) and its size might change through a clinical application of live three-dimensional ultrasound imaging.

In live three-dimensional imaging of the heart with a matrix transducer, that is a two-dimensional array transducer, there is a need for high volume acquisition rates to be able to properly visualize the dynamic structures of the heart. At present, one means of achieving high volume rates is to employ 4X multi-line imaging or so-called parallel receive beamforming. In this, four receive beams are simultaneously formed in a symmetric pattern around a single transmit beam. Multiple sets of these patterns scan across the volume to acquire the volumetric image data. This method depends on the transmit beam being broad enough in area to illuminate each of the receive beams that surround it. Because the volume rate is determined by the number of acoustic scanning lines in each volume, receiving four scanning lines simultaneously increases the volume rate by a factor of four compared to the simple case of one receive scanning line for each transmit beam. In practice with acoustic imaging and, even, with 4X multi-line imaging, the acoustic lines, specifically the receive lines, can only be spread out so far before problems are encountered.

The first problem is that as the received beams move apart, they also move away from the transmit beam that is illuminating those receive beams. Hence, the image looses sensitivity and becomes dim. This can be helped commonly by increasing the width of the transmit beam by reducing the transmit aperture and/or reducing the transmit frequency. Another common way to help is to increase the width of the receive beams by reducing the receive aperture. Both techniques demonstrate some improvement but without sufficient benefit to maintain sufficient high volume rates for live three-dimensional imaging in case the region of interest is a large volume.

The second problem that is encountered when spreading the receive lines out is that at some point there are gaps between the receive lines and targets that are between the lines, in particular at a greater depth, are missed and significant spatial aliasing occurs. Enlarging the receive beams by reducing the receive apertures helps, but again does not provide sufficient benefit.

Reference US 4,442,713 A discloses an ultrasonic imaging apparatus having an array of transducer elements for transmitting ultrasonic signals in an object to be analyzed through a use of the transmitted signals reflected from the object and sensed by the apparatus. Adjusting the number of transmitting and/or receiving transducers with changes in frequency produced by signal attenuation is suggested to improve the image resolution under a wider variety of used conditions.

Document US 2009/203996 discloses a 3D ultrasound imaging system, which permits the operator to selectively choose a number of display-mode parameters that result in an operator directed view of the volume-of-interest. Spatial density may be influenced by operator inputs, where the variations in spatial-line density are used to minimize acoustic-echo acquisition time or to optimize resolution in specific regions of the target volume.

Document GB 1262694 discloses an apparatus for the directional transmission and reception of sound waves in water. The turning of the central frequency of the bandpass filter used in this apparatus is inversely proportional to the distance of the acoustic centres of the ultrasound transducers.

There is a need to further improve such three-dimensional ultrasound systems.

### SUMMARY OF THE INVENTION

It is an object of the present invention to provide an improved ultrasound system and method. It is a further object of the present invention to provide a computer program for implementing such method.

In a first aspect of the present invention an ultrasound imaging system for providing a three-dimensional image of a volume is presented. The ultrasound imaging system comprises a transducer array configured to provide an ultrasound receive signal, a beam former configured to control the transducer array to scan the volume along a multitude of scanning lines, and further configured to receive the ultrasound receive signal and to provide an image signal, a signal processor configured receive the image signal and to conduct a bandpass filtering operation around a central receive frequency on the image signal, wherein the signal processor is further configured to adjust the central receive frequency as a function of a spacing of the scanning lines, wherein the signal processor is configured to lower the central receive frequency when the spacing is increased, wherein the signal processor is further configured to provide image data, an image processor configured to receive the image data from the signal processor and to provide display data, and a display configured to receive the display data and to provide the three-dimensional image.

In a further aspect of the present invention a method for providing of a three-dimensional image of a volume is presented. The method comprises the steps of scanning the volume along a multitude of scanning lines with a transducer array, receiving a signal from the transducer array, processing the signal by conducting a bandpass filtering operation on the signal to provide image data, wherein a central receive frequency of the bandpass filtering operation is adjusted as a function of a spacing of the scanning lines, wherein the central receive frequency is lowered when the spacing increases, and displaying the three-dimensional image using the image data.

In a further aspect of the invention a computer program comprising program code means for causing a computer, in particular an ultrasound imaging system, to carry out the steps of such method when said computer program is carried out on a computer.

The basic idea of the invention is to reduce the central receive frequency of a bandpass filter of the signal processor as a function of increasing line spacing.

In the clinical application of live three-dimensional imaging, there is sometimes a need to image a relatively small area of the heart such as a single valve, or a septal defect, and there is sometimes the need to image a large area of the heart such as the entire left ventricle. In need of both cases there is a need to maintain a sufficiently high volume rate, for example 20Hz or at least 24 Hz. When changing between the large area case and the small area case, there is willingness on the part of the clinician to decrease the imaging resolution when imaging large areas and to increase the resolution when imaging smaller areas. This willingness allows an ultrasound system to maintain high volume rates across both small and large area imaging by maintaining a fixed number of acoustic lines and, hence, a fixed volume rate, regardless of the size of the volume being imaged. It has been found out that if the line spacing is varied to maintain a sufficiently high volume acquisition rate when the size of the volume to be inspected or the region of interest is enlarged, reducing the receive frequency and, further, bandwidth as a function of increasing line spacing allows for much greater separation between lines without significant loss in sensitivity and increase in spatial aliasing.

It is implemented in the signal processor to shift the receive frequency and, further, bandwidth of the bandpass filters as a function of the given line spacing. The line spacing in turn varies when the size of the volume to be inspected is changed.

Preferred embodiments of the invention are defined in the dependent claims. It shall be understood that the claimed method has similar and/or identical preferred embodiments as the claimed device and as defined in the dependent claims.

In an embodiment, the signal processor is configured to adjust the central receive frequency based on a linear relationship between the spacing and the central receive frequency. As an alternative, the signal processor is configured to adjust the central receive frequency based on a non-linear relationship between the spacing and the central receive frequency. It has been found that a relationship as simple as a linear relationship between the receive frequency and the line spacing is sufficient to allow a greater separation of acoustic lines without a loss in sensitivity and spatial aliasing.

In a further embodiment, the non-linear relationship between the central receive frequency and the spacing is a polynomial function. In particular, in a further embodiment, the polynomial function is a second-order polynomial function of the form

$$SF = 1 - A \cdot \left(LS - MLS\right)^2,$$

wherein SF is a receive frequency shift factor, LS is the spacing in degrees, MLS is a minimum line spacing in degrees and A is a scaling parameter. By this, the relatively simple implementation of a second-order relationship between the shifting factor and the line spacing can be provided. In particular, no shifting occurs when the line spacing is the minimum line spacing. Then, however, with increasing line spacing, the shifting factor becomes lower in a progressive fashion. In practice there has been found that a relationship such that the reduction in receive frequency starts out slowly as the line spacing increases and then increases in rate as the line spacing increases works well.

In a further embodiment, the non-linear relationship between the central receive frequency and the spacing is an exponential function. In particular, the exponential function is of the form

$$SF = 1 - A \cdot \left(LS - MLS\right) \cdot B^{LS},$$

wherein SF is a shift factor of the central receive frequency, LS is the spacing in degrees and MLS is a minimum line spacing in degrees, A is a scaling parameter and B is a scaling parameter. In practice, it has been found that an exponential relationship such that the reduction in receive frequency starts out slowly as the line spacing increases then increases in rate as the line spacing increases further works well.

Furthermore, the relationships are implemented in the signal processor with a set of parameters that give the user control of the relationship between line spacing and the shift factor.

In a further embodiment, the signal processor is further configured to adjust a bandwidth of the bandpass filtering operation as a function of the spacing of the scanning lines, specifically the receive scanning lines, wherein the signal processor is configured to lower the bandwidth when the spacing is increased. It has been found out that decreasing not only the central receive frequency of the bandpass filtering operation but also the bandwidth further increases the possibility to increase the spacing between the scanning lines without a loss in sensitivity and an increase in spatial aliasing.

In a further embodiment, the signal processor is further configured to adjust the bandwidth and the central receive frequency by a same shift factor. This allows for a simpler configuration of the signal processor while maintaining the advantageous technical effects.

In a further embodiment, the bandpass filter is quadrature bandpass filter. Such a bandpass filter allows for a good signal processing and, in particular, an easy implementation of the shifting of the central receive frequency and the bandwidth.

BRIEF DESCRIPTION OF THE DRAWINGS

These and other aspects of the invention will be apparent from and elucidated with reference to the embodiment(s) described hereinafter. In the following drawings

Fig. 1 shows a schematic illustration of an ultrasound system according to an embodiment;
Fig. 2a shows a schematic representation of a region of interest in relation to an ultrasonic probe;
Fig. 2b shows a schematic example how a multitude of scanning lines may spread through the volume in Fig. 2a;
Fig. 3a shows an illustration of a bandpass filtering operation;
Fig. 3b shows a first embodiment of a frequency shift of a bandpass filtering operation;
Fig. 3c shows a second embodiment of a frequency shift of a bandpass filtering operation;
Fig. 4 shows a schematic block diagram of the ultrasound system according to the embodiment;
Fig. 5 shows an example for a relationship between line space in degrees and a shift factor;
Fig. 6a shows an illustrative example of a first image without frequency shifting;
Fig. 6b shows an illustrative example of a second image with applied frequency shifting; and
Fig. 7 shows a schematic flow diagram of a method according to an embodiment.

DETAILED DESCRIPTION OF THE INVENTION

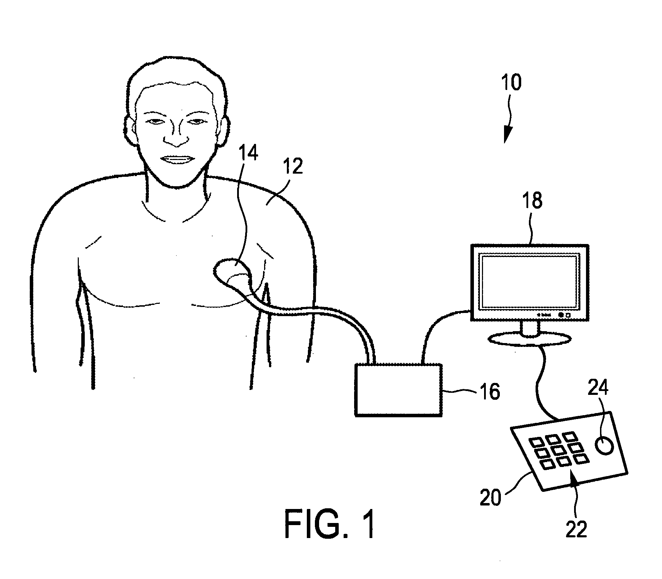

Fig. 1 shows a schematic illustration of an ultrasound system 10 according to an embodiment, in particular a medical ultrasound three-dimensional imaging system. The ultrasound system 10 is applied to inspect a volume of an anatomical site, in particular an anatomical site of a patient 12. The ultrasound system 10 comprises an ultrasound probe 14 having at least one transducer array having a multitude of transducer elements for transmitting and/or receiving ultrasound waves. In one example, the transducer elements each can transmit ultrasound waves in form of at least one transmit impulse of a specific pulse duration, in particular a plurality of subsequent transmit pulses. The transducer elements can for example be arranged in a one-dimensional row, for example for providing a two-dimensional image that can be moved or swiveled around an axis mechanically. Further, the transducer elements may be arranged in a two-dimensional array, in particular for providing a multi-planar or three-dimensional image. In particular, the transducer array is used with parallel receive beamforming, i.e. a multitude of receive beams is illuminated by a single "fat" transmit beam.

In general, the multitude of two-dimensional images, each along a specific acoustic line or scanning line, in particular scanning receive line, may be obtained in three different ways. First, the user might achieve the multitude of images via manual scanning. In this case, the ultrasound probe may comprise position-sensing devices that can keep track of a location and orientation of the scan lines or scan planes. However, this is currently not contemplated. Second, the transducer may be automatically mechanically scanned within the ultrasound probe. This may be the case if a one dimensional transducer array is used. Third, and preferably, a phased two-dimensional array of transducers is located within the ultrasound probe and the ultrasound beams are electronically scanned. The ultrasound probe may be hand-held by the user of the system, for example medical staff or a doctor. The ultrasound probe 14 is applied to the body of the patient 12 so that an image of an anatomical site in the patient 12 is provided.

Further, the ultrasound system 10 has a controlling unit 16 that controls the provision of a three-dimensional image via the ultrasound system 10. As will be explained in further detail below, the controlling unit 16 controls not only the acquisition of data via the transducer array of the ultrasound probe 14 but also signal and image processing that form the three-dimensional images out of the echoes of the ultrasound beams received by the transducer array of the ultrasound probe 14.

The ultrasound system 10 further comprises a display 18 for displaying the three-dimensional images to the user. Further, an input device 20 is provided that may comprise keys or a keyboard 22 and further inputting devices, for example a track ball 24. The input device 20 might be connected to the display 18 or directly to the controlling unit 16.

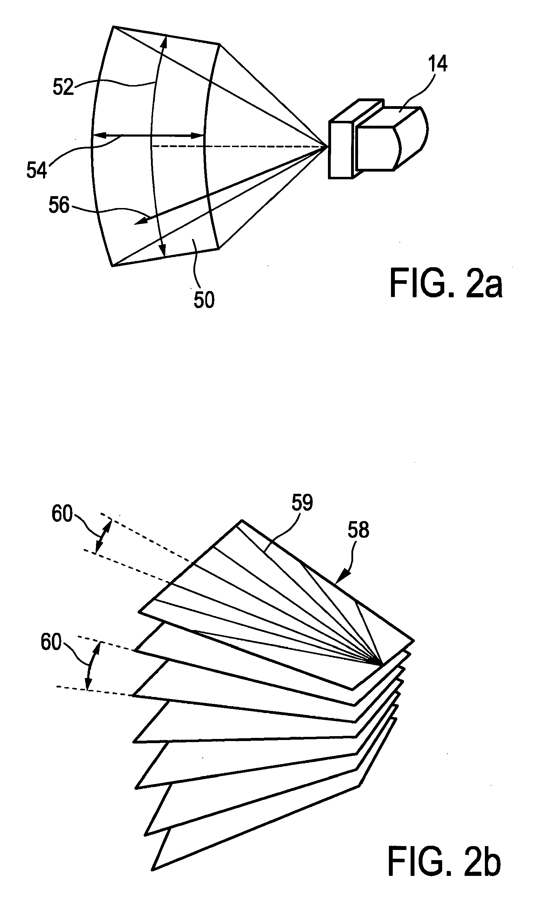

Fig. 2a shows an example of a volume 50 relative to the ultrasound probe 14. The exemplary volume 50 depicted in this example is of a sector type, due to the transducer array of the ultrasound probe 14 being arranged as a phased two-dimensional electronically scanned array. Hence, the size of the volume 50 may be expressed by an elevation angle 52 and a lateral angle 54. A depth 56 of the volume 50 may be expressed by a so-called line time in seconds per line. That is the scanning time spent to scan a specific scanning line.

Fig. 2b shows an illustrative example how the volume 50 may be divided into a multitude of slices 58 or two-dimensional images each acquired along a multitude of so-called scan lines 59. During image acquisition, the two-dimensional transducer array of the ultrasound probe 14 is operated by a beam former in a way that the volume 50 is scanned along a multitude of these scan lines 58 sequentially. However, in multi-line receive processing, a single transmit beam might illuminate a multitude, for example four, receive scanning lines along which signals are acquired in parallel. If so, such sets of receive lines are then electronically scanned across the volume 50 sequentially.

Hence, a resolution of a three-dimensional image processed out of the acquired two-dimensional images depends on a so-called line density which in turn depends on a spacing 60 between two adjacent scanning lines 59. In fact, it is the distance between two adjacent scan lines 59 within a slice 58 and, further, between the slices 58. As a result, the line density in the direction of the lateral extent and in the direction of the elevation extent is the same. Hence, the line density is measured in the form of degrees per line.

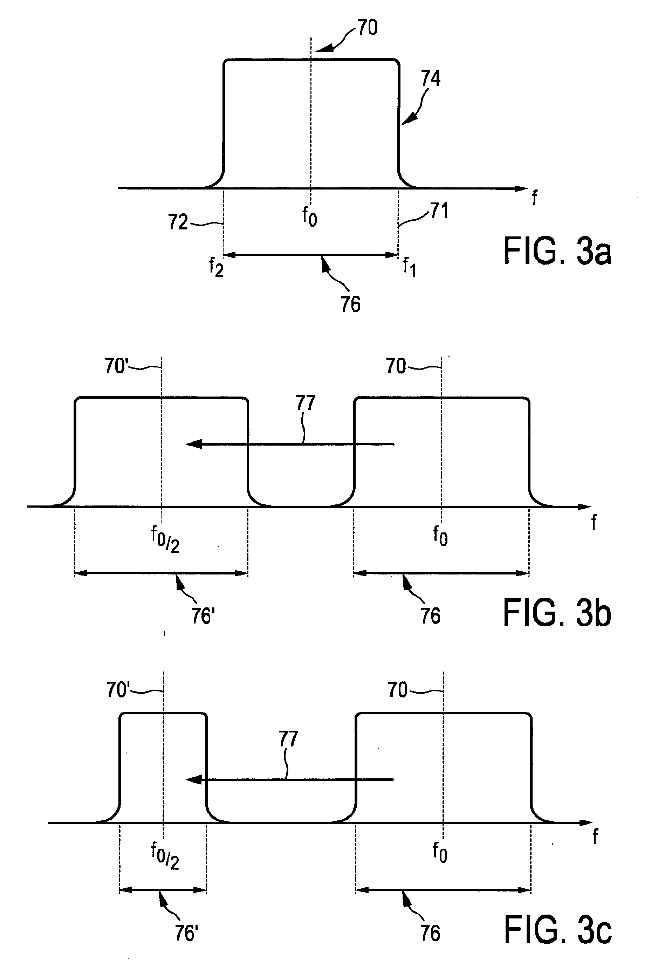

Fig. 3a shows an illustration of a bandpass filtering operation. A bandpass filtering operation occurs around a central receive frequency 70. The bandpass occurs between an upper frequency 71 and a lower frequency 72. A signal 74 on which the bandpass filtering operation is supplied, only passes between the upper frequency 71 and the lower frequency 72. Parts of the signal 74 above the upper frequency 71 and below the lower frequency 72 are cut off. Hence, the signal 74 only passes in a bandwidth 76 around the central receive frequency 70.

Fig. 3b shows a first embodiment of a frequency shift of the bandpass filtering operation. The frequency shift is schematically depicted by an arrow 77. This embodiment, only the central receive frequency 70 is shifted to a central receive frequency 70'. In the depicted embodiment, the central receive frequency 70 is halved to the central receive frequency 70. Hence, the bandwidth in which the signal 74 passes is altered from a bandwidth 76 to a bandwidth 76'.

Fig. 3c shows a second embodiment of a frequency shift of a bandpass filtering operation. In this embodiment, not only the central receive frequency 70 but also the bandwidth is reduced as the line spacing increases. Hence, the central receive frequency 70 is lowered by half to the central receive frequency 70'. Further, the bandwidth 76 is lowered to a bandwidth 76' which is half of the bandwidth 76. Therefore, the central receive frequency 70 and the bandwidth 76 are scaled with the same shift factor.

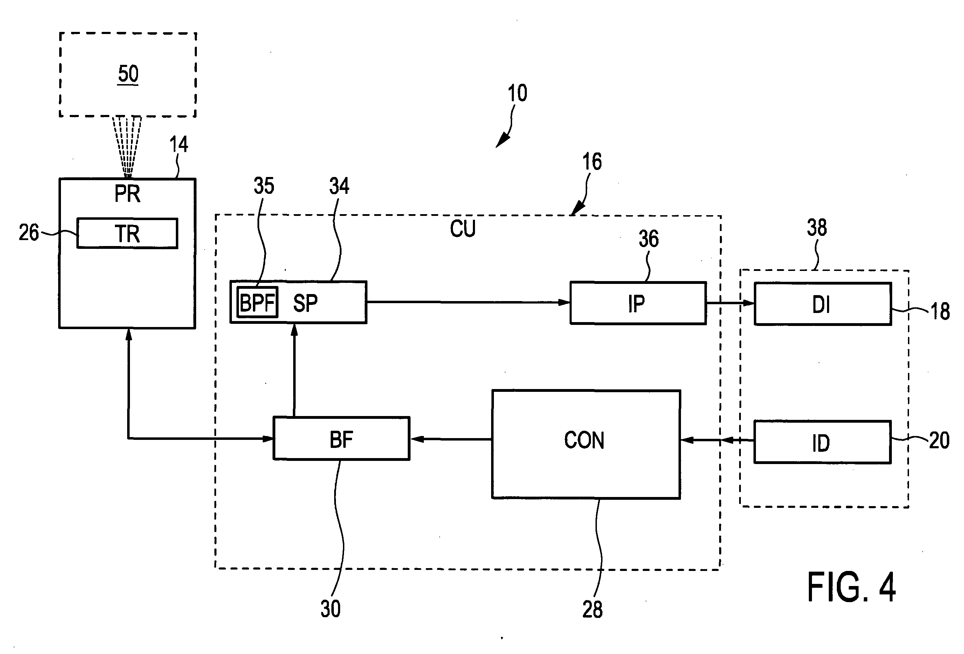

Fig. 4 shows a schematic block diagram of the ultrasound system 10. As already laid out above, the ultrasound system 10 comprises an ultrasound probe (PR) 14, the controlling unit (CU) 16, the display (DI) 18 and the input device (ID) 20. As further laid out above, the probe 14 comprises a phased two-dimensional transducer array 26. In general, the controlling unit (CU) 16 may comprise a central processing unit that may include analog and/or digital electronic circuits, a processor, microprocessor or the like to coordinate the whole image acquisition and provision. Further, the controlling unit 16 comprises a herein called image acquisition controller 28. However, it has to be understood that the image acquisition controller 28 does not need to be a separate entity or unit within the ultrasound system 10. It can be a part of the controlling unit 16 and generally be hardware or software implemented. The current distinction is made for illustrative purposes only.

The image acquisition controller 28 as part of the controlling unit 16 may control a beam former and, by this, what images of the volume 50 are taken and how these images are taken. The beam former 30 generates the voltages that drives the transducer array 26, determines parts repetition frequencies, it may scan, focus and apodize the transmitted beam and the reception or receive beam(s) and may further amplify filter and digitize the echo voltage stream returned by the transducer array 26. Further, the controller 28 of the controlling unit 16 may determine general scanning strategies. Such general strategies may include a desired volume acquisition rate, lateral extent of the volume, an elevation extent of the volume, maximum and minimum line densities, scanning line times and the line density as already explained above.

The beam former 30 further receives the ultrasound signals from the transducer array 26 and forwards them as image signals.

Further, the ultrasound system 10 comprises a signal processor 34 that receives the image signals. The signal processor 34 is generally provided for analogue-to-digital-converting, digital filtering, for example, band pass filtering, as well as the detection and compression, for example a dynamic range reduction, of the received ultrasound echoes or image signals. The signal processor forwards image data. In particular, the signal processor 34 comprises a bandpass filter 35. The bandpass filter 35 may be a quadrature bandpass filter.

The quadrature bandpass filter 35 provides three functions. First, a band limiting of the image signal. Second, producing in-phase and quadrature pairs of scan line data and, third, digitally demodulating echo-signals to an intermediate or baseband range of frequencies. The characteristics by the quadrature bandpass filter are determined by parameters inputted by the controlling unit 16. Into the controlling unit 16, the parameters might be user input via the user interface 38. This enables the user to control the relationship between line spacing and frequency shift. The relationship between frequency shift and bandwidth and the line spacing is such that the central receive frequency and bandwidth are lowered when the line spacing increases. This is in such way that the reduction of the receive frequency and bandwidth starts out slowly as the line spacing increases and then increases in rate as the line spacing increases. Possible examples are a second-order polynomial function of the form

$$SF = 1 - A \cdot (LS - MLS)^2$$

wherein SF is a receive frequency shift factor, LS is the spacing in degrees, MLS is a minimum line spacing in degrees and A is a scaling parameter, and an exponential function of the form

$$SF = 1 - A \cdot (LS - MLS) \cdot B^{LS}$$

wherein SF is a shift factor of the central receive frequency, LS is the spacing in degrees and MLS is a minimum line spacing in degrees, A is a scaling parameter and B is a scaling parameter may be applied.

By this, large line spacing can be applied without loss in sensitivity. Hence, the large line spacing allows for maintaining a total number of acoustic lines within the volume to be inspected, even if the volume is large. By maintaining the total number of acoustic lines and increasing the spacing between the acoustic lines, even larger volumes can be inspected with the ultrasound system while maintaining the volume acquisition rate and, therefore, enable an acquisition rate highly enough to provide live three-dimensional ultrasound imaging.

Further, the ultrasound system 10 comprises an image processor 36 that converts image data received from the signal processor 34 into display data finally shown on the display 18. In particular, the image processor 36 receives the image data, preprocesses the image data and may store it in an image memory. These image data is then further post-processed to provide images most convenient to the user via the display 18. In the current case, in particular, the image processor 36 may form the three-dimensional images out of a multitude of two-dimensional images acquired along the multitude of scan lines 59 in each slice 58.

A user interface is generally depicted with reference numeral 38 and comprises the display 18 and the input device 20. It may also comprise further input devices, for example, a mouse or further buttons which may even be provided on the ultrasound probe 14 itself.

A particular example for a three-dimensional ultrasound system which may apply the current invention is the CX50 CompactXtreme Ultrasound system sold by the applicant, in particular together with a X7-2t TEE transducer of the applicant or another transducer using the xMATRIX technology of the applicant. In general, matrix transducer systems as found on Philips iE33 systems or mechanical 3D/4D transducer technology as found, for example, on the Philips iU22 and HD15 systems may apply the current invention.

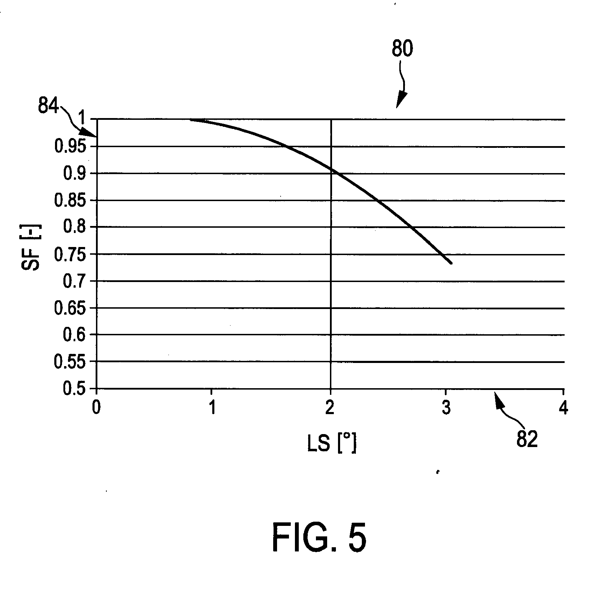

Fig. 5 shows an example diagram 80 having an x-axis showing a line spacing in degrees and a y-axis showing the corresponding shift factor. The graph starts out at a minimum line spacing, in this case 0.75 degrees and ends at a maximum line spacing, in this case 3 degrees. Such graph could be used implemented the relationship between line spacing and central receive frequency 70. The decrease in the shift factor starts out slowly from the minimum line spacing and increases a rate as the line spacing increases. A possible second-order polynomial that provides a corresponding graph could be

$$SF = 1 - 0,051 \cdot (LS - 0,75)^2 .$$

Further, the following exponential relationship could be used to provide a similar graph:

$$SF = 1 - 0,00574 \cdot (LS - 0,75) \cdot e^{LS} .$$

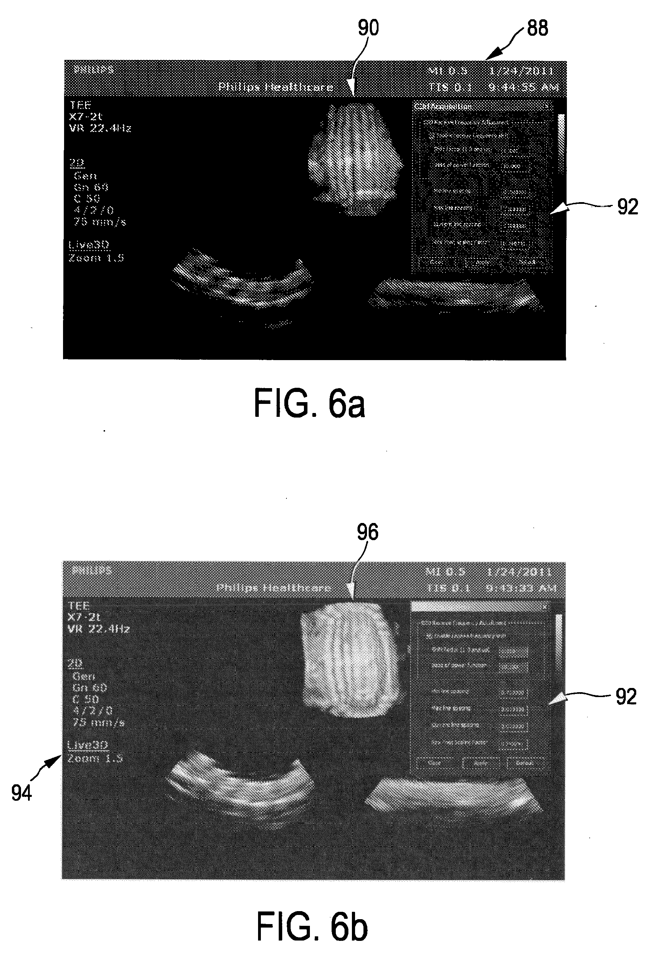

Fig. 6a shows an illustrative example of a first display. The first display shows a first image 90 acquired without frequency shifting. As shown by reference numeral 92, a dialog box 92 may be provided via the display 18 so that a user may selectively enable or disable the receive frequency shift. Further, second parameters can be inputted via the input device 20, for example, a base for an exponential function other parameters of the relationship between line spacing and shift factor. Further, a minimum line spacing and a maximum line spacing may be inputted by a user.

In contrast, Fig. 6b shows a second display 94 giving a second image 96 of the same volume with enabled central receive frequency shift according to the invention. As is clearly visible, the quality of the picture is significantly enhanced.

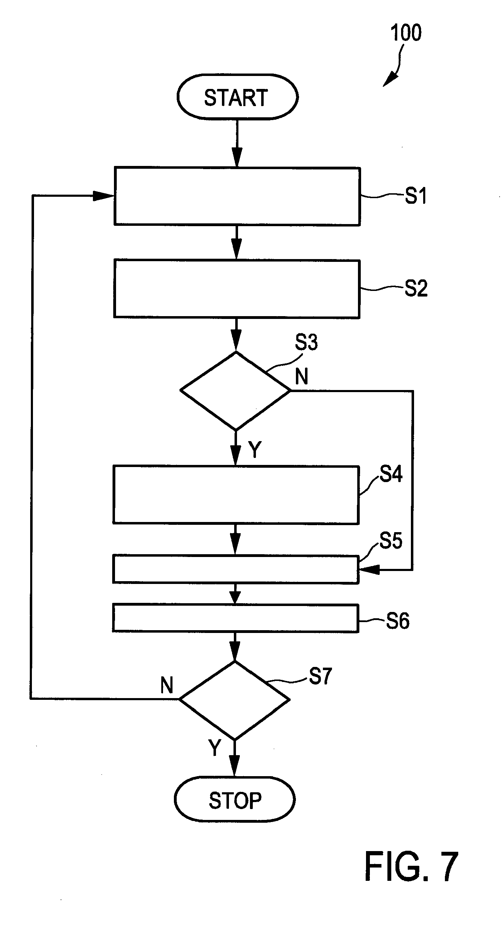

Fig. 7 shows an embodiment of a method. After the method has started, the volume is scanned along a multitude of scanning lines 59, specifically a multitude of receive scanning lines, with a transducer array 26. In a further step S2 a signal is received from the transducer array 26. In particular, the transducer array 26 may forward ultrasound signals to the beam former 30. The beam former 30 in turn converts the ultrasound signals to image signals which are then forwarded to the signal processor 34.

In a step S3, it is determined that the line spacing is changed. If not, in a step S5, a bandpass filtering operation is applied on the signal to provide image data and, further, in a step S6, a three-dimensional image is displayed using the image data.

However, if in step S3 is determined that the line spacing is changed, the method continues with a step S4 in which a central receive frequency 70 of the bandpass filtering operation is adjusted as a function of a spacing of the scanning lines. In particular, the adjustment can be conducted according to one of the relationships or formulas given above. Further, the bandwidth 76 of the bandpass filtering operation may be lowered with same shift factor.

Then, again in step S5, the bandpass filtering operation is conducted to provide image data and, in step S6, this three-dimensional image is displayed using the image data.

Last, in step S7, it is determined whether the scanning operation ends. If so, the method ends. If not, the method starts over in step S1.

While the invention has been illustrated and described in detail in the drawings and foregoing description, such illustration and description are to be considered illustrative or exemplary and not restrictive; the invention is not limited to the disclosed embodiments.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. An ultrasound imaging system (10) for providing a three-dimensional image of a volume (50), the ultrasound imaging system comprising:

   a transducer array (26) configured to provide an ultrasound receive signal,
   a beam former configured to control the transducer array to scan the volume (50) along a multitude of scanning lines (59), and further configured to receive the ultrasound receive signal and to provide an image signal,
   a signal processor (34) configured to receive the image signal and to conduct a bandpass filtering operation around a central receive frequency (70) on the image signal,
   wherein the signal processor is further configured to provide image data,
   an image processor (36) configured to receive the image data from the signal processor (34) and to provide display data, and
   a display (18) configured to receive the display data and to provide the three-dimensional image,

   **characterised in that** the signal processor (34) is further configured to adjust the central receive frequency as a function of a spacing (60) of the scanning lines (59), wherein the signal processor is configured to lower the central receive frequency when the spacing is increased.

2. The system of claim 1, wherein the signal processor is configured to adjust the central receive frequency based on a linear relationship between the spacing and the central receive frequency.

3. The system of claim 1, wherein the signal processor is configured to adjust the central receive frequency based on a non-linear relationship between the spacing and the central receive frequency.

4. The system of claim 3, wherein the non-linear relationship between the central receive frequency and the spacing is a polynomial function.

5. The system of claim 4, wherein the polynomial function is a second-order polynomial function of the form

$$SF = 1 - A \cdot \left(LS - MLS\right)^2$$

wherein SF is a receive frequency shift factor, LS is the spacing (60) in degrees, MLS is a minimum line spacing in degrees and A is a scaling parameter.

6. The system of claim 3, wherein the non-linear relationship between the central receive frequency and the spacing is an exponential function.

7. The system of claim 6, wherein the exponential function is of the form

$$SF = 1 - A \cdot \left(LS - MLS\right) \cdot B^{LS}$$

wherein SF is a shift factor of the central receive frequency, LS is the spacing (60) in degrees and MLS is a minimum line spacing in degrees, A is a scaling parameter and B is a scaling parameter.

8. The system of claim 1, wherein the signal processor (34) is further configured to adjust a bandwidth of the bandpass filtering operation as a function of the spacing (60) of the scanning lines (59), wherein the signal processor is configured to reduce the bandwidth when the spacing is increased.

9. The system of claim 8, wherein the signal processor (34) is further configured to adjust the bandwidth and the central receive frequency by a same shift factor.

10. The system of claim 1, wherein the bandpass filter is a quadrature bandpass filter.

11. A method for providing of a three-dimensional ultrasound image of a volume (50), the method comprising the following steps:

scanning (S1) the volume along a multitude of scanning lines (59) with a transducer array (26), receiving (S2) a signal from the transducer array (26),
processing (S5) the signal by conducting a bandpass filtering operation on the signal to provide image data, wherein a central receive frequency (70) of the bandpass filtering operation is adjusted (S4) as a function of a spacing (60) of the scanning lines (59), wherein the central receive frequency is lowered when the spacing increases, and
displaying (S6) the three-dimensional image using the image data.

12. Computer program comprising program code means for causing a computer to carry out the steps of the method as claimed in claim 11 when said computer program is carried out on a computer.

**Patentansprüche**

1. Ultraschallbildgebungssystem (10) zur Bereitstellung eines dreidimensionales Bild eines Volumens (50), wobei das Ultraschallbildgebungssystem Folgendes umfasst:

ein Wandlerarray (26), das dafür eingerichtet ist, ein Ultraschallempfangssignal bereitzustellen;
einen Strahlformer, der dafür eingerichtet ist, das Wandlerarray so zu steuern, dass das Volumen (50) entlang einer Vielzahl von Abtastlinien (59) abgetastet wird, und der weiterhin dafür eingerichtet ist, das Ultraschallempfangssignal zu empfangen und ein Bildsignal bereitzustellen;
einen Signalprozessor (34), der dafür eingerichtet ist, das Bildsignal zu empfangen und das Bildsignal um eine zentrale Empfangsfrequenz (70) herum einer Bandpassfilterung zu unterziehen, wobei der Signalprozessor weiterhin dafür eingerichtet ist, Bilddaten bereitzustellen;
einen Bildprozessor (36), der dafür eingerichtet ist, die Bilddaten von dem Signalprozessor (34) zu empfangen und Anzeigedaten bereitzustellen; und
eine Anzeige (18), die dafür eingerichtet ist, die Bilddaten zu empfangen und das dreidimensionale Bild bereitzustellen;
**dadurch gekennzeichnet, dass** der Signalprozessor (34) weiterhin dafür eingerichtet ist, die zentrale Empfangsfrequenz in Abhängigkeit vom Abstand (60) der Abtastlinien (59) anzupassen, wobei der Signalprozessor dafür eingerichtet ist, die zentrale Empfangsfrequenz zu verringern, wenn sich der Abstand vergrößert.

2. System nach Anspruch 1, wobei der Signalprozessor dafür eingerichtet ist, die zentrale Empfangsfrequenz basierend auf einem linearen Zusammenhang zwischen dem Abstand und der zentralen Empfangsfrequenz anzupassen.

3. System nach Anspruch 1, wobei der Signalprozessor dafür eingerichtet ist, die zentrale Empfangsfrequenz basierend auf einem nicht-linearen Zusammenhang zwischen dem Abstand und der zentralen Empfangsfrequenz anzupassen.

4. System nach Anspruch 3, wobei der nicht-lineare Zusammenhang zwischen der zentralen Empfangsfrequenz und dem Abstand eine Polynomfunktion ist.

5. System nach Anspruch 4, wobei die Polynomfunktion eine Polynomfunktion zweiter Ordnung in der Form

$$SF = 1 - A \cdot (LS - MLS)^2$$

ist, wobei SF ein Empfangsfrequenz-Verschiebungsfaktor ist, LS der Abstand (60) in Grad ist, MLS ein Mindestlinienabstand in Grad ist und A ein Skalierungsparameter ist.

6. System nach Anspruch 3, wobei der nicht-lineare Zusammenhang zwischen der zentralen Empfangsfrequenz und dem Abstand eine Exponentialfunktion ist.

7. System nach Anspruch 6, wobei die Exponentialfunktion in der Form

$$SF = 1 - A \cdot (LS - MLS) \cdot B^{LS}$$

vorliegt, wobei SF Verschiebungsfaktor der zentralen Empfangsfrequenz ist, LS der Abstand (60) in Grad ist, MLS ein Mindestlinienabstand in Grad ist, A ein Skalierungsparameter ist und B ein Skalierungsparameter ist.

8. System nach Anspruch 1, wobei der Signalprozessor (34) weiterhin dafür eingerichtet ist, eine Bandbreite der Bandpassfilterung in Abhängigkeit vom Abstand (60) der Abtastlinien (59) anzupassen, wobei der Signalprozessor dafür eingerichtet ist, die Bandbreite zu verringern, wenn sich der Abstand vergrößert.

9. System nach Anspruch 8, wobei der Signalprozessor (34) weiterhin dafür eingerichtet ist, die Bandbreite und die zentrale Empfangsfrequenz durch den gleichen Verschiebungsfaktor anzupassen.

10. System nach Anspruch 1, wobei der Bandpassfilter ein Quadratur-Bandpassfilter ist.

11. Verfahren zur Bereitstellung eines dreidimensionales Bilds eines Volumens (50), wobei das Verfahren die folgenden Schritte umfasst:

Abtasten (S1) des Volumens entlang einer Vielzahl von Abtastlinien (59) mit einem Wandlerarray (26);
Empfangen (S2) eines Signals von dem Wandlerarray (26);
Verarbeiten (S5) des Signals durch Durchführen einer Bandpassfilterung für das Signal, um Bilddaten bereitzustellen, wobei eine zentrale Empfangsfrequenz (70) der Bandpassfilterung in Abhängigkeit vom Abstand (60) der Abtastlinien (59) angepasst (S4) wird, wobei die zentrale Empfangsfrequenz verringert wird, wenn sich der Abstand vergrößert; und
Anzeigen (S6) des dreidimensionalen Bilds unter Verwendung der Bilddaten.

12. Computerprogramm mit Programmcodemitteln zum Veranlassen eines Computers, die Schritte des Verfahrens nach Anspruch 11 durchzuführen, wenn das genannte Computerprogramm auf einem Computer ausgeführt wird.

**Revendications**

1. Système d'imagerie ultrasonore (10) pour fournir une image tridimensionnelle d'un volume (50), le système d'imagerie ultrasonore comprenant :

un réseau de transducteurs (26) configuré pour fournir un signal de réception ultrasonore,
un dispositif de mise en forme de faisceau configuré pour commander au réseau de transducteurs de balayer le volume (50) le long d'une multitude de lignes de balayage (59), et configuré en outre pour recevoir le signal de réception ultrasonore et pour fournir un signal d'image,
un processeur de signal (34) configuré pour recevoir le signal d'image et pour effectuer une opération de filtrage passe-bande autour d'une fréquence de réception centrale (70) sur le signal d'image, dans lequel le processeur de signal est en outre configuré pour fournir des données d'image,
un processeur d'image (36) configuré pour recevoir les données d'image du processeur de signal (34) et pour fournir des données d'affichage, et
un affichage (18) configuré pour recevoir les données d'affichage et pour fournir l'image tridimensionnelle,
**caractérisé en ce que** le processeur de signal (34) est en outre configuré pour ajuster la fréquence de réception centrale en fonction d'un espacement (60) des lignes de balayage (59), dans lequel le processeur de signal est configuré pour réduire la fréquence de réception centrale lorsque l'espacement est augmenté.

2. Système selon la revendication 1, dans lequel le processeur de signal est configuré pour ajuster la fréquence de réception centrale sur la base d'une relation linéaire entre l'espacement et la fréquence de réception centrale.

3. Système selon la revendication 1, dans lequel le processeur de signal est configuré pour ajuster la fréquence de réception centrale sur la base d'une relation non linéaire entre l'espacement et la fréquence de réception centrale.

4. Système selon la revendication 3, dans lequel la relation non linéaire entre la fréquence de réception centrale et l'espacement est une fonction polynomiale.

5. Système selon la revendication 4, dans lequel la fonction polynomiale est une fonction polynomiale de deuxième ordre de la forme :

$$SF = 1 - A \cdot (LS - MLS)^2$$

où SF est un facteur de décalage de fréquence de réception, LS est l'espacement (60) en degrés, MLS est un espacement de lignes minimal en degrés et A est un paramètre de mise à l'échelle.

6. Système selon la revendication 3, dans lequel la relation non linéaire entre la fréquence de réception centrale et l'espacement est une fonction exponentielle.

7. Système selon la revendication 6, dans lequel la fonction exponentielle est de la forme :

$$SF = 1 - A \cdot (LS - MLS) \cdot B^{LS}$$

où SF est un facteur de décalage de fréquence de réception centrale, LS est l'espacement (60) en degrés, MLS est un espacement de lignes minimal en degrés, A est un paramètre de mise à l'échelle et B est un paramètre de mise à l'échelle.

8. Système selon la revendication 1, dans lequel le processeur de signal (34) est en outre configuré pour ajuster une largeur de bande de l'opération de filtrage passe-bande en fonction de l'espacement (60) des lignes de balayage (59), dans lequel le processeur de signal est configuré pour réduire la largeur de bande lorsque l'espacement est augmenté.

9. Système selon la revendication 8, dans lequel le processeur de signal (34) est en outre configuré pour ajuster la largeur de bande et la fréquence de réception centrale d'un même facteur de décalage.

10. Système selon la revendication 1, dans lequel le filtre passe-bande est un filtre passe-bande en quadrature.

11. Procédé pour fournir une image ultrasonore tridimensionnelle d'un volume (50), le procédé comprenant les étapes suivantes :

le balayage (S1) du volume le long d'une multitude de lignes de balayage (59) avec un réseau de transducteurs (26),
la réception (S2) d'un signal du réseau de transducteurs (26),
le traitement (S5) du signal en effectuant une opération de filtrage passe-bande sur le signal pour fournir des données d'image, dans lequel une fréquence de réception centrale (70) de l'opération de filtrage passe-bande est ajustée (S4) en fonction d'un espacement (60) des lignes de balayage (59), dans lequel la fréquence de réception centrale est réduite lorsque l'espacement est augmenté, et

l'affichage (S6) de l'image tridimensionnelle en utilisant les données d'image.

12. Programme informatique comprenant des moyens de code de programme pour amener un ordinateur à effectuer les étapes du procédé selon la revendication 11 lorsque le programme informatique est exécuté sur un ordinateur.

FIG. 1

52

54

56

50

14

FIG. 2a

60

60

59

58

FIG. 2b

**FIG. 3a**

**FIG. 3b**

**FIG. 3c**

FIG. 4

FIG. 5

FIG. 6a

FIG. 6b

100

FIG. 7

## REFERENCES CITED IN THE DESCRIPTION

**Patent documents cited in the description**

- US 4442713 A
- US 2009203996 A

- GB 1262694 A